Europäisches Patentamt

European Patent Office

Office européen des brevets

(19)

(11) EP 0 775 089 B1

(12) EUROPEAN PATENT SPECIFICATION

(45) Date of publication and mention
of the grant of the patent:
**28.04.1999 Bulletin 1999/17**

(51) Int Cl.[6]: **C02F 1/32**, A61L 2/10,
B01D 29/01, B01D 29/66

(21) Application number: **95928549.5**

(22) Date of filing: **11.08.1995**

(86) International application number:
**PCT/GB95/01905**

(87) International publication number:
**WO 96/05141 (22.02.1996 Gazette 1996/09)**

(54) **FLUID TREATMENT APPARATUS**

VORRICHTUNG ZUR BEHANDLUNG VON FLÜSSIGKEITEN

APPAREIL DE TRAITEMENT DE FLUIDE

(84) Designated Contracting States:
**BE DE DK ES FR IT NL PT SE**

(30) Priority: **11.08.1994 GB 9416287**

(43) Date of publication of application:
**28.05.1997 Bulletin 1997/22**

(73) Proprietor: **WATER RECOVERY PLC
Bicester, Oxon OX6 9JT (GB)**

(72) Inventor: **SNOWBALL, Malcolm, Robert
Epping, Essex CM16 6TW (GB)**

(74) Representative: **Evans, Huw David Duncan
Urquhart-Dykes & Lord,
Three Trinity Court,
21-27 Newport Road
Cardiff CF2 1AA (GB)**

(56) References cited:
**EP-A- 0 316 687          WO-A-94/06482
DE-A- 3 247 747**

- **PATENT ABSTRACTS OF JAPAN vol. 008 no.
067 (C-216) ,29 March 1984 & JP,A,58 220000
(TOKYO SHIBAURA DENKI KK) 21 December
1983,**

**Description**

This invention relates to fluid treatment apparatus and more particularly, but not solely, to an apparatus for treating drinking water.

Drinking water may be contaminated with parasitic micro-organisms such as Cryptosporidium and Giardias, which are particularly harmful to human beings if ingested. These micro-organisms are extremely difficult to treat by conventional means, since they are resistant to chlorine and other biocides. They are also relatively resistant to ultra violet radiation owing to their thick outer membranes. However, it is known that the micro-organisms are susceptible to extremely high doses of ultra-violet radiation.

Conventional ultra-violet fluid treatment apparatus, such as the one disclosed in GB 2175779, comprise an elongate tubular chamber having an axially extending ultra-violet light source. The dose of radiation supplied by the apparatus is equal to the product of the radiation intensity of the light source and the retention time of the fluid inside the chamber. A high fluid flow rate is needed, but it is impractical to increase substantially the radiation intensity of the light.

We have now devised a fluid treatment apparatus which is able to effectively treat Cryptosporidium and Giardia parasitic micro-organisms but which can maintain a high fluid flow rate.

In accordance with this invention there is provided a fluid treatment apparatus comprising a flow passage, a plurality of filter elements mounted across the flow passage for filtering fluid flowing along the passage between an inlet and an outlet of the passage, backwashing means for reversing the flow of fluid through a said filter element whilst fluid flows along the passage between said inlet and outlet through at least one other said filter element, and radiating means for irradiating a surface of the or each said other filter element so as to kill any trapped micro-organisms, the backwashing means being arranged to reverse the flow of fluid through each filter element in succession whilst fluid flows along the passage between said inlet and outlet through at least one other said filter element.

In use, any micro-organisms trapped by the filter are exposed to a continuous dose of radiation, which is sufficiently large to ensure that the micro-organisms are killed. Each filter is backwashed in succession, so as to remove any dead micro-organisms or other debris which may have built up thereon. At least one filter is arranged across the flow passage during backwashing of another filter, so that untreated micro-organisms are unable to pass through the apparatus.

Preferably the apparatus is arranged such that a predetermined delay occurs between reversing the flow of fluid through adjacent filter elements in the flow passage. Thus ensures that all of the micro-organisms receive at least the minimum amount of radiation required to kill them.

Cryptosporidium and Giardia micro-organisms are 4-6 microns in diameter, therefore the filter preferably comprises a filter material having pore sizes which are less than 4 microns.

Preferably the radiating means comprises an ultra-violet light source. Preferably the light source radiates short-wave ultra-violet light having a wavelength of 245-265nm, and particularly 253.7nm. Micro-organisms are most susceptible to radiation at these wavelengths. Preferably the radiating means irradiates opposite surfaces of the filter.

Thus, the flow of fluid does not need to be interrupted in order to perform backwashing.

In one embodiment the flow passage comprises a chamber divided into two longitudinally extending portions by a filter, each portion of the chamber being provided with respective fluid inlet and outlet ports, said backwashing means comprising valves connected to said inlet and outlet ports, and control means for selectively opening the inlet port of one portion of the chamber and the outlet port of the other portion of the chamber, so as to cause fluid flow in one direction through the filter. In order to reverse the flow of fluid through the filter the inlet and outlet valves of the opposite portions of the chamber are opened instead. It will be appreciated that fluid continues flow between an inlet and an outlet of the chamber regardless of the direction of fluid flow through the filter.

In an alternative embodiment the filter is rotatably mounted in the fluid flow passage, said backwashing means comprising an actuator which rotates the filter through 180°, so that fluid flows through the filter in the reverse direction.

Preferably the filters are arranged in series.

In one embodiment, the filter comprises a mesh formed from titanium. When water contacts the filter it forms a titanium dioxide film on the surface of the mesh. This film reacts with the short-wave UV light to produce hydroxyls in the oxide surface. If a micro-organism comes into contact with this activated surface it is killed, owing to the oxidisation of its cell wall.

In an alternative embodiment, the filter comprises a fibrous material which is transparent to radiation emitted by said radiation means.

Preferably the fibrous material comprises PTFE, which is transparent to short-wave UV light.

Embodiments of this invention will now be described by way of examples only, and with reference to the accompanying drawings, in which:

FIGURE 1 is a sectional view through an embodiment of water treatment apparatus in accordance with this invention; and
FIGURE 2 is a sectional view through an alternative embodiment of water treatment apparatus in accordance with this invention.

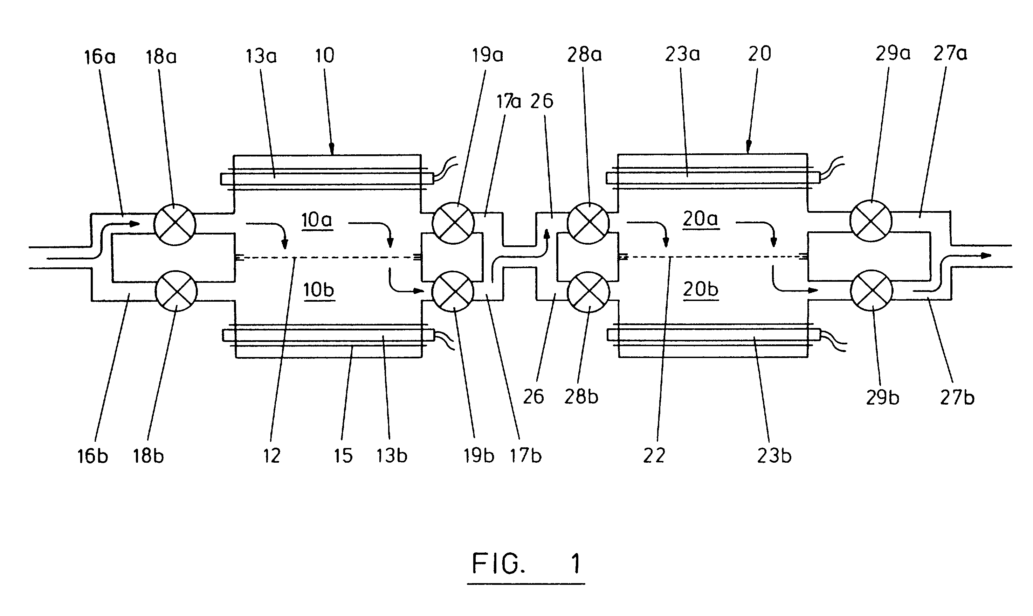

Referring to Figure 1 of the drawings, there is shown a water treatment apparatus comprising two elongate chambers 10,20 connected in series in a water flow passage. The first chamber 10 comprises upper and lower, longitudinally extending, portion 10a,10b separated by a titanium mesh filter 12. A pair of elongate ultra-violet lamps 13a,13b extend through the upper and lower portions 10a,10b of the chamber respectively. Each ultra-violet lamp e.g. 13b is mounted inside a quartz glass sleeve 15, which is sealed at its opposite ends to the walls of the chamber.

The upper and lower portions 10a,10b of the chamber are connected at one end to respective branches 16a,16b of an inlet duct, and at the other end to respective branches 17a,17b of an outlet duct. The upper and lower branches of the inlet and outlet ducts 16a,16b,17a,17b are provided with respective fluid flow valves 18a,18b,19a,19b.

The second chamber 20 also comprises upper and lower longitudinally extending portions 20a,20b separated by a titanium mesh filter 22. A pair of elongate ultra-violet lamps 23a,23b extend through the upper and lower portions 20a,20b of the chamber respectively.

The upper and lower branches 17a,17b of the outlet duct of the first chamber 10 are each connected to upper and lower branches 26a,26b of an inlet duct to the second chamber 20. The upper and lower portions 20a,20b of the second chamber are connected at one end to the respective branches 26a,26b of the inlet duct, and at the other end to respective branches 27a,27b of an outlet duct. The upper and lower branches of the inlet and outlet ducts 26a,26b,27a,27b to the second chamber are provided with respective fluid flow valves 28a, 28b,29a,29b.

In use, the valves 18a,19b are opened, such that water flows into the upper portion 10a of the first chamber, through the first filter 12 and into the lower portion 10b of the chamber. The valves 28a,29b are also opened such that water flowing out of the lower portion 10b of chamber flows into the upper portion 20a of the second chamber, through the second filter 22, into the lower portion 20b of the chamber, and out of the apparatus through the outlet duct 27b. Assuming that the ultra-violet lamps 13a,13b,23a,23b are illuminated, then any Cryptosporidium or Giardia micro-organisms in the water will be caught by the first filter 12, and held in front of the radiation from the lamps 13a,13b.

Assuming that the minimum time which the micro-organisms are held in front of the ultra-violet radiation is 5 minutes and that the lamps produce a minimum of 4mW/cm$^2$ of ultra-violet radiation, then the dose will be:

$$\text{Dose} = 4 \times (5 \times 60)$$

$$= 1200 \text{ mW Sec/cm}^2.$$

It is known that a dose of 50-100 mW Sec/cm$^2$

kills Cryptosporidium and Giardia micro-organisms. Thus, the apparatus in accordance with this invention irradiates the micro-organisms with a dose which is 12 times greater than is needed to kill them. Also, there is very little build up of other debris on the filter, since the apparatus is intended for use in treating clean drinking water.

After 5 minutes the valves 18b,19a are opened, and the valves 18a,19b closed, so that water flows in the reverse direction through the first filter 12, thereby backwashing the filter. Any debris and micro-organisms caught by the first filer 12 are washed through onto the second filter 22, where they receive a further dose of radiation. After 5 more minutes the valves 28b,29a are opened, and the valves 28a,29b closed, so that water now flows in the reverse direction through the second filter.

All the harmful micro-organisms on the second filter 22 have been killed, and thus the second filter can be safely backwashed into the flow stream out of the apparatus.

This process continues, so that the direction of flow through each filter 12,22 is reversed every 10 minutes, with a 5 minute interval between successive reversals, so that all micro-organisms caught by the apparatus are exposed to radiation for at least 5 minutes.

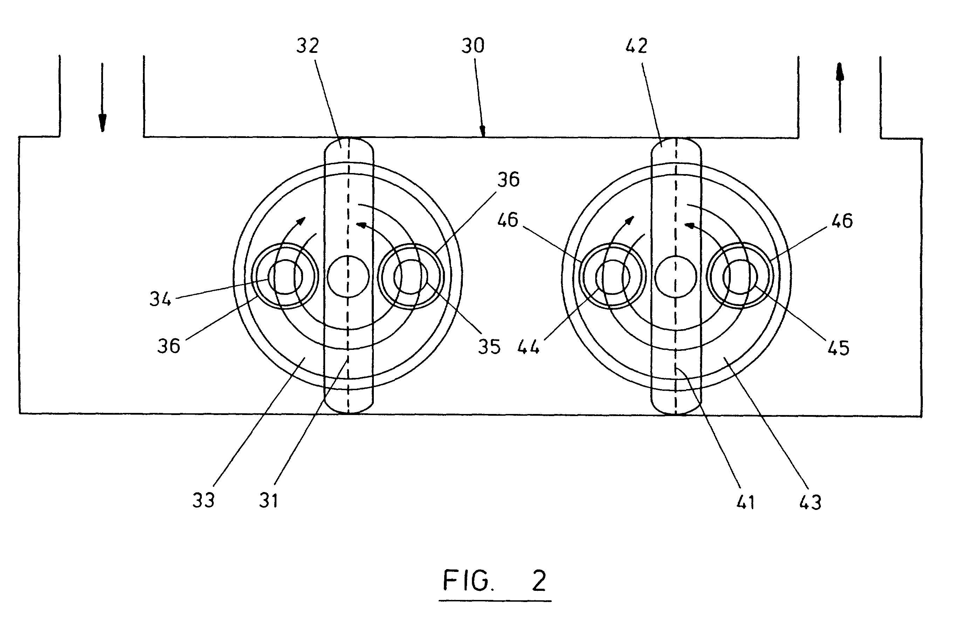

Referring to Figure 2 of the drawings, there is shown an alternative embodiment of water treatment apparatus comprising an elongate tubular chamber 30 connected in series with a water flow passage. A circular titanium mesh filter 31, having an annular seal 32 around its periphery, is mounted normal to the direction of fluid flow, such that all of the water flowing through the apparatus is filtered. The filter 31 is mounted on a rotatable carrier 33, which also carries a pair of tubular ultra-violet lamps 34,35 mounted inside quartz glass sleeves 36. The lamps 34,35 are arranged to illuminate opposite sides of the filter 31.

A second titanium mesh filter 41, having an annular seal 42 around its periphery, is mounted downstream of the first filter 31. The second filter 41 is mounted on another rotatable carrier 43, which also carries a pair of tubular ultra-violet lamps 44,45 mounted inside quartz glass sleeves 46.

In use, micro-organism are trapped by the filter 31, which is mounted across the flow passage. The ultra-violet lamps 34,35 illuminate the filter. After 5 minutes the carrier 33 is rotated through 180°, so that water now flows in the reverse direction through the filter 31, thereby backwashing the filter. Any debris and micro-organisms caught by the first filter 31 are washed through onto the second filter 41, where they receive a further dose of radiation from the lamps 44,45. After 5 more minutes the carrier 43 is rotated, so that water now flows in the reverse direction through the second filter 41.

As before, all the harmful micro-organisms which have collected on the filter 41 are killed by the ultra-violet radiation, and thus the debris from the second filter can be safely backwashed into the flow out of the apparatus.

Each filter is rotated through 180° every 10 minutes, with a 5 minute interval between successive rotations, so that all of the micro-organisms caught by the filter are exposed to radiation for at least 5 minutes.

It will be appreciated that both embodiments of the invention deliver a dose of radiation to all of the trapped micro-organisms, which is at least 12 times greater than is necessary to kill them. The mesh filters used do not significantly affect the water flow rate, so that there is little resulting loss in water pressure at the consumer. The effectiveness of the system can be increased by providing more than two filters.

## Claims

1. A fluid treatment apparatus comprising a flow passage, a plurality of filter elements mounted across the flow passage for filtering fluid flowing along the passage between an inlet and an outlet of the passage, backwashing means for reversing the flow of fluid through a said filter element whilst fluid flows along the passage between said inlet and outlet through at least one other said filter element, and radiating means for irradiating a surface of the or each said other filter element so as to kill any trapped micro-organisms, the backwashing means being arranged to reverse the flow of fluid through each filter element in succession whilst fluid flows along the passage between said inlet and outlet through at least one other said filter element.

2. A fluid treatment apparatus as claimed in claim 1, arranged so that a predetermined minimum delay occurs between reversing the flow of fluid through adjacent filter elements in the flow passage.

3. A fluid treatment apparatus as claimed in claims 1 or 2, in which the filter element comprises a mesh of titanium.

4. A fluid treatment apparatus as claimed in claims 1 or 2, in which the filter element comprises a fibrous material which is transparent to radiation emitted by said radiating means.

5. A fluid treatment apparatus as claimed in claim 4, in which the fibrous material comprises PTFE.

6. A fluid treatment apparatus as claimed in any preceding claims, in which the radiating means comprises an ultra-violet light source.

7. A fluid treatment apparatus as claimed in claim 6, in which the light source radiates ultra-violet light having a wavelength of 245-265 nM.

8. A fluid treatment apparatus as claimed in claim 7, in which the light source radiates ultra-violet light having a wavelength of 253.7 nM.

9. A fluid treatment apparatus as claimed in any preceding claims, in which the flow passage comprises a pair of chambers each divided into two longitudinally extending portions by respective filter elements, each portion of each chamber being provided with respective fluid inlet and outlet ports, said backwashing means comprising valves connected to said ports and control means for selectively opening the inlet ports of one portion of each of the chambers and the outlet ports of the other portions of the respective chambers.

10. A fluid treatment apparatus as claimed in any of claims 1 to 8, in which each filter is rotatably mounted in the fluid flow passage, said backwashing means comprising actuators which rotate the filters through 180°, so that fluid flows through the latter in the reverse direction.

## Patentansprüche

1. Vorrichtung zur Behandlung von Fluiden mit einem Strömungskanal, mehreren quer über den Strömungskanal angebrachten Filterelementen zum Filtern von Fluid, das zwischen einem Eingang und einem Ausgang des Strömungskanals den Kanal entlangströmt, Rückspülmittel zur Umkehrung des Fluidstroms durch eines der Filterelemente, während Fluid zwischen dem Eingang und dem Ausgang durch mindestens ein anderes Filterelement den Strömungskanal entlangfließt, und Bestrahlungsmitteln zum Bestrahlen einer Oberfläche des bzw. jedes anderen Filterelements zur Abtötung von eingefangenen Mikroorganismen, wobei die Rückspülmittel so angeordnet sind, daß sie den Fluidstrom durch jedes Filterelement nacheinander umkehren, während Fluid zwischen dem Eingang und dem Ausgang durch mindestens ein anderes Filterelement den Strömungskanal entlangfließt.

2. Vorrichtung zur Behandlung von Fluiden nach Anspruch 1, die so ausgelegt ist, daß zwischen der Umkehrung des Fluidstroms durch nebeneinanderliegende Filterelemente im Strömungskanal eine vorgegebene Mindestverzögerung auftritt.

3. Vorrichtung zur Behandlung von Fluiden nach Anspruch 1 oder 2, bei der das Filterelement aus einem Titannetz besteht.

4. Vorrichtung zur Behandlung von Fluiden nach Anspruch 1 oder 2, bei der das Filterelement aus einem für die von den Bestrahlungsmitteln ausgesandte Strahlung durchlässigen Fasermaterial besteht.

5. Vorrichtung zur Behandlung von Fluiden nach Anspruch 4, bei der es sich bei dem Fasermaterial um PTFE handelt.

6. Vorrichtung zur Behandlung von Fluiden nach einem der vorhergehenden Ansprüche, bei der es sich bei dem Bestrahlungsmittel um eine UV-Lichtquelle handelt.

7. Vorrichtung zur Behandlung von Fluiden nach Anspruch 6, bei der die Lichtquelle ultraviolettes Licht mit einer Wellenlänge von 245-265 nm aussendet.

8. Vorrichtung zur Behandlung von Fluiden nach Anspruch 7, bei der die Lichtquelle ultraviolettes Licht mit einer Wellenlänge von 253,7 nm aussendet.

9. Vorrichtung zur Behandlung von Fluiden nach einem der vorhergehenden Ansprüche, bei der der Strömungskanal zwei Kammern umfaßt, die jeweils durch zugehörige Filterelemente in zwei längliche Teile aufgeteilt werden, welche jeweils mit zugehörigen Fluideingangs- und -ausgangsöffnungen versehen sind, wobei die Rückspülmittel mit den Öffnungen verbundene Ventile und Steuermittel zur selektiven Öffnung der Eingangsöffnungen eines Teils einer jeden der Kammern und der Ausgangsöffnungen der anderen Teile der jeweiligen Kammern enthalten.

10. Vorrichtung zur Behandlung von Fluiden nach einem der Ansprüche 1 bis 8, bei der jedes Filter im Strömungskanal drehbar angebracht ist, wobei die Rückspülmittel Stellglieder enthalten, die die Filter um 180° drehen, so daß diese in umgekehrter Richtung von Fluid durchströmt werden.

**Revendications**

1. Appareil de traitement de fluide comprenant un passage d'écoulement, une pluralité d'éléments de filtre montés en travers du passage d'écoulement pour filtrer le fluide s'écoulant le long du passage entre une entrée et une sortie du passage, un moyen de lavage à contre-courant pour inverser l'écoulement de fluide à travers undit élément de filtre tandis que le fluide s'écoule le long du passage entre lesdites entrée et sortie à travers au moins un autre dit élément de filtre, et un moyen d'irradiation pour irradier une surface du ou de chacun desdits autres éléments de filtre de manière à détruire tout micro-organisme prisonnier, le moyen de lavage à contre-courant étant arrangé pour inverser l'écoulement de fluide à travers chaque élément de filtre en succession tandis que le fluide s'écoule le long du passage entre lesdites entrée et sortie à travers au moins un autre dit élément de filtre.

2. Appareil de traitement de fluide selon la revendication 1, disposé de telle sorte qu'il se produise un retard minimum prédéterminé entre l'inversion de l'écoulement de fluide à travers des éléments de filtre adjacents dans le passage d'écoulement.

3. Appareil de traitement de fluide selon les revendications 1 ou 2, dans lequel l'élément de filtre comprend une maille en titane.

4. Appareil de traitement de fluide selon les revendications 1 ou 2, dans lequel l'élément de filtre comprend un matériau fibreux qui est transparent aux rayonnements émis par ledit moyen d'irradiation.

5. Appareil de traitement de fluide selon la revendication 4, dans lequel le matériau fibreux comprend du PTFE.

6. Appareil de traitement de fluide selon l'une quelconque des revendications précédentes, dans lequel le moyen d'irradiation comprend une source de lumière ultra-violette.

7. Appareil de traitement de fluide selon la revendication 6, dans lequel la source de lumière émet de la lumière ultra-violette ayant une longueur d'onde de 245-265 nm.

8. Appareil de traitement de fluide selon la revendication 7, dans lequel la source de lumière émet de la lumière ultra-violette ayant une longueur d'onde de 253,7 nm.

9. Appareil de traitement de fluide selon l'une quelconque des revendications précédentes, dans lequel le passage d'écoulement comprend une paire de chambres divisées chacune en deux portions s'étendant longitudinalement par des éléments de filtre respectifs, chaque portion de chaque chambre étant pourvue d'orifices d'entrée et de sortie de fluide respectifs, ledit moyen de lavage à contre-courant comprenant des soupapes connectées auxdits orifices et un moyen de commande pour ouvrir de manière sélective les orifices d'entrée d'une portion de chacune des chambres et les orifices de sortie des autres portions des chambres respectives.

10. Appareil de traitement de fluide selon l'une quelconque des revendications 1 à 8, dans lequel chaque filtre est monté à rotation dans le passage d'écoulement de fluide, ledit moyen de lavage à contre-courant comprenant des actionneurs qui font tourner les filtres de 180°, de sorte que du fluide s'écoule à travers ces derniers dans la direction inverse.

FIG. 1

FIG. 2